# EUROPEAN PATENT SPECIFICATION

(11) **EP 1 885 319 B1**
(45) Date of publication and mention of the grant of the patent: **14.11.2012**
(21) Application number: 05800949.9
(22) Date of filing: 22.09.2005
(51) Int. Cl.: A61N 7/00, A61H 23/02

(54) **NEOPLASM CELL DESTRUCTION DEVICE**
VORRICHTUNG ZUR ZERSTÖRUNG VON NEOPLASIEN
DISPOSITIF DE DESTRUCTION DE CELLULES NEOPLASIQUES

(30) Priority: 20.05.2005 US 134011
(43) Date of publication of application: 13.02.2008
(73) Proprietor: Kenny, Daniele J., Miller Place, NY 11764 (US)
(72) Inventor: Kenny, Daniele J., Miller Place, NY 11764 (US)
(74) Representative: Luchs, Willi
(86) International application number: PCT/US2005/033978
(87) International publication number: WO 2006/130168

(56) References cited:
- WO-A2-00/15097
- US-A- 4 315 514
- US-A- 4 343 301
- US-A- 4 747 142
- US-A- 5 413 550
- US-A- 5 501 655

## Description

### Background of the Invention

### Field of the Invention:

The present invention relates to a cell destruction device, and more particularly, the present invention relates to a neoplasm cell destruction device.

### Description of the Prior Art:

Ultrasound may be used to remotely heat industrial or biological materials . There has been strong evidence in research and clinical laboratories that focused ultrasound for cancer hyperthermia will become a useful mode of treating cancer patients, in addition to the surgical, radiological, and chemotherapeutic methods available now.

In the treatment of tumors in cancer hyperthermia, focused ultrasound heats the tumor to a temperature of approximately 43<0>C, while the adjacent healthy tissue is kept at a lower temperature, closer to normal body temperature (37<0>C.). The elevated temperature in the tumor disrupts the tumor growth and eventually kills it. This allows the cancer to potentially be treated without surgery, without ionizing radiation, or without chemotherapy.

U.S. Patent No. 4,343,301 teaches a method of generating a high energy density, noninvasively, by two high frequency sonic beams creating a low frequency beating pattern at their intersection locus. One method provides for two transducers at different angular positions. Each transducer produces a beam pattern of high frequency. One transducer produces a high frequency which is higher by a predetermined quantity than the other. At their point of intersection, the sonic oscillations add and subtract, producing a low frequency beat equal to the predetermined quantity. This high energy low frequency beat can be used to stimulate neural points in the skull or other parts of the body or for tissue destruction. But it needs a pair of transducers, which work just on one point and unsuitable for destruction of neoplasm cell.

Conventional focused ultrasound for heating is employed by using either a scanned ultrasound transducer or a phased array. The scanned transducer uses a lens, much like an optical magnifying glass focus sunlight, while the phased array uses electronic delays among the array elements to achieve focusing. A burst of ultrasound is then emitted converging at the focus to provide localized high intensity energy. Some of the high energy is absorbed by the tissue at the focus and is dissipated as concentrated focal heat. The rest of the energy travels through the focus and is slowly dissipated into the surrounding tissues as distributed heat.

Biomedical hyperthermia applicators using a plurality of ultrasound sources to heat larger, distributed volumes, have also been investigated. These investigations have relied upon linear thermal superposition of the plurality of ultrasound sources to heat the target tissue. Nonlinear effects of ultrasound propagation through animal tissue and materials have also been studied for a single ultrasound source.

It is generally recognized that the use of microwave energy to produce moderate internal heating is also an effective tool in the treatment of tissue, especially neoplastic tumors. The primary factor limiting such treatment in the past has been the difficulty of delivering the heat to a target region below the skin surface without causing cavitation and/or heating of healthy soft tissues.

In order to achieve significant heating in tumors more than a few millimeters below the skin surface, the field from a single source at the skin surface will have to be high and therefore painful. See U.S. Patent Number 5,503,150 to Evans at col. 1, lines 26-29.

One approach has used a moving source, generally activated by switching discrete sub-arrays of sources. The moving ultrasound source, however, results in an incoherent summation of energy at the tumor site. While tending to reduce the heating effects in the intervening tissue, this method has not eliminated the heating of the intervening tissue or reduced it to an acceptable level.

Additionally with ultrasound therapy, to insure that the desired volume of tissue is potentially heated, an operator must not only know the characteristics in the area of interest, but also be able to determine which tissues are being heated. Currently, the ability to make this determination depends on the use of an interstitial probe or a radiometer. The current method also does not allow for imaging of the area, except to use other modalities, such as CT.

Further, it has been found that cancer cells are rapidly killed by mechanical trauma associated with shape-transitions causing increases in cell surface area. The hypothesis has been advanced that these increases in surface area occur in two phases. First, there is an apparent increase as a result of surface unfolding, which is reversible and therefore non-lethal. Second, there is a true increase during which cell surface membranes are stretched, with an increase in membrane tension. When tension exceeds a critical level, the surface membranes rupture and the irreversible change is lethal to the cell. See L. Weiss, J.P. Harlos, and G. Elkin; Int. J. Cancer 44; 143-148 (1989).

Numerous other innovations for wave treatments have been provided in the prior art that will be described infra. Even though these other innovations may be suitable for the specific individual purposes to which they address, however, they differ from the present invention.

For example, U.S. Patent No. 3,880,152 issued to Nohmura on April 29, 1975 teaches a chair or a bed with speakers incorporated therein. The speakers are disposed against the inside surfaces of the seat and back of the chair, and the top surface of the bed, so that the openings of the speakers will be directed toward a human body resting therein.

Another example, U.S. Patent No. 4,055,170 issued to Nohmura on October 25, 1977 teaches a health promoting apparatus including a chair, bed, or the like with a loudspeaker incorporated therein. An opening formed in the chair is closed by a pretensioned flexible sheet. The sound waves from the loudspeaker cause the flexible sheet to vibrate thereby transmitting vibrations to a chair occupant.

Still another example, U.S. Patent No. 4,315,514 issued to Drewes et al. on February 16, 1982 teaches an ultrasound apparatus and a method for destroying selected cells in a host without damage to non-selected cells including selecting a transmission path from an energy source to the selected cells, determining one or more of the resonant frequencies of the selected cells, selecting as a destructive frequency one of the resonant frequencies at which the transmissibility of the selected cells is higher than the transmissibility of the non-selected cells in the transmission path, and transmitting energy from the source at the destructive frequency along the path with sufficient intensity to destroy the selected cells without destroying the non-selected cells.

Yet another example, U.S. Patent No. 4,674,505 issued to Pauli et al. teaches an essentially planar shock wave generated with the assistance of a shock wave tube via a magnetic dynamic effect. The shock wave is focused by an acoustic convergent lens, whereby the calculus to be pulverized is placed at the focal point of the convergent lens. In order to couple the shock wave to the patient, the space that the shock wave traverses is filled with a coupling agent, for example water. The shock wave tube, the convergent lens, and a fine adjustment for the displacement of the convergent lens relative to the shock wave tube are attached to a mounting stand so as to be pivotable in all directions. The disintegration facility includes a shock wave tube having high operating reliability with respect to high voltage, requires low maintenance, and has only negligible imaging or focusing errors resulting from the shock wave producing membrane and the convergent lens.

Still yet another example, U.S. Patent No. 4,753,225 issued to Vogel on June 28, 1988 teaches therapy equipment for the human body serving to enhance the feeling of good health by exposure of a part of or all of the body to acoustic irradiation with frequencies in the sub-audio, audio, and ultrasonic regions. The therapy equipment includes at least one oscillator plate arranged in bodily contact with the body of the person who sits, lies, or stands on it. The oscillator plate is made to oscillate by sound waves, whereby corresponding oscillation generators are secured in bodily contact to the oscillator plate. The frequency of the sound waves is adjusted to the reabsorption frequency of individually selected organs and parts of the body to treat selective individual organs or parts of the human body.

Yet still another example, U.S. Patent No. 5,062,412 issued to Okazaki on November 5, 1991 teaches electrically and simultaneously forming a plurality of focused regions of shock waves. A shock wave generating apparatus includes a plurality of high-voltage pulse generators for generating a plurality of high-voltage pulses, a shock wave generating unit having a plurality of ultrasonic vibrating element groups coupled to the plurality of high voltage pulse generators for generating shock waves and for focusing the shock waves onto a plurality of different focused regions within a biological body under examination, and a plurality of delay units coupled via the high-voltage pulse generators to the plural ultrasonic vibrating element groups for causing the plurality of high-voltage pulses having predetermined delay times to be generated from the high-voltage pulse generators whereby the plural focused regions are simultaneously formed juxtaposed each other near a concretion to be disintegrated with the biological body.

Still yet another example, U.S. Patent No. 5,086,755 issued to Schmid-Eilber on February 11, 1992 teaches a chaise lounge for therapeutic treatment of a patient including three support sections hinged together so as to be pivotable relative to one another for comfortably supporting a patient. The support sections have openings formed therein spaced along the longitudinal centerline of the chaise lounge and electroacoustic transducers movably disposed below the openings and adapted to radiate upwardly through the openings at the lower back, the chest, and the head/neck areas of a patient resting on the chaise longue with an enhanced signal of a frequency corresponding to the rhythm frequency of certain music to which the patient's body is exposed. The rhythm frequency is in the non-audible range and adapted to achieve total relaxation of the patient.

Yet still another example, U.S. Patent No. 5,095,890 issued to Houghton et al. on March 17, 1992 teaches a method for automatically optimizing ultrasonic frequency power applied by a transducer to human tissue while the transducer is energized with ultrasonic signals from an ultrasonic signal generator. The frequency of an ultrasonic energizing signal applied by the ultrasonic signal generator to the transducer is set. The frequency of the energizing signal applied to the ultrasonic signal generator to the transducer is scanned at reoccurring intervals through a sequence of frequencies. The optimum level of power from the transducer is monitored as the frequency is scanned. The frequency of the ultrasonic energizing signal applied by the ultrasonic signal generator is ultimately reset substantially at the frequency causing the optimum level of power until the next reoccurring interval.

Still yet another example, U.S. Patent No. 5,143,063 issued to Fellner on September 1, 1992 teaches an electromedical apparatus employed to non-invasively remove adipose tissue from the body by causing necrosis thereof by localizing,- e.g. focusing, radiant energy. The radiant energy may be of any suitable kind, for example, localized radio frequency, microwave, or ultrasound energy that is impinged upon the cells to be eliminated. Cell destruction occurs through a mechanism such as, e.g., heating or mechanical disruption beyond a level that the adipose tissue can survive.

Yet still another example, U.S. Patent No. 5,144,953 issued to Wurster et al. on September 8, 1992 teaches a lithotriptor with an X-ray alignment system including a transducer for generating focused ultrasonic shock waves adapted for alignment on a concretion or tissue to be destroyed. The transducer is connected to an image-forming diagnostic X-ray system for locating the concretion or tissue and includes an X-ray emitter and an image intensifier disposed on a pivotable frame. The transducer is connected to the X-ray emitter that in turn is disposed at the center of the transducer so that the emission axes of the transducer and the X-ray emitter coincide.

Still yet another example, U.S. Patent No. 5,178,134 issued to Vago on January 12, 1993 teaches ultrasonic treatment of animals. The equipment is able to apply ultrasonic waves with at least two power densities in the vicinity of the portion of the animal, with one of the power densities being more than 15 watts per square meter for sterilizing the water before the patient enters the tube and the other being less than 15 watts per square meter.

Yet still another example, U.S. Patent No. 5,209,221 issued to Riedlinger on May 11, 1993 teaches a device for generating sonic signal forms for limiting, preventing, or regressing the growth of pathological tissue including an ultrasonic transmission system for transmitting sound waves focused on the tissue to be treated by way of a coupling medium. An ultrasonic signal produced at the focus of the system includes brief pulses having at least one rarefaction phase with a negative sonic pressure amplitude with a value greater than 2 x 10⁵ Pa. The ultrasonic signal is radiated with a carrier frequency exceeding 20 kHz, a sonic pulse duration T of less than 100 µs and a pulse recurrence rate of less than 1/(5T). The device produces controlled cavitation in the tissue to be treated.

Still yet another example, U.S. Patent No. 5,222,484 issued to Krauss et al. on June 29, 1993 teaches an apparatus for shock wave treatment including a shock wave transducer with a cup-shaped body and with an X-ray location finding device for finding the location of a bodily concretion or tissue to be treated. The X-ray device includes an extendable X-ray tube with telescoping tube sections sealed against an acoustic coupling medium filling the delay path of the transducer by a balloon filling arranged within the X-ray tube. The balloon is secured to the upper section of the tube and to the lower section thereof. Over pressure or under pressure is applied to the interior of the X-ray tube to adjust its length in order to optimize X-ray location finding on the one hand and shock wave treatment on the other hand.

Yet still another example, U.S. Patent No. 5,388,581 issued to Bauer et al. on February 14, 1995 teaches a therapy apparatus for treating concretions and tissue in the body of a patient by way of sound waves. The apparatus includes a sound wave generator and an available X-ray device for locating an object for therapy. The therapy apparatus has a spot film device arranged within the axial passage of an X-ray cone. The available X-ray device is attached to the sound wave generator, with its central longitudinal axis aligned with the focus thereof so as to be able to precisely adjust and fix the X-ray device to the therapy apparatus quickly and safely.

Still yet another example, U.S. Patent No. 5,435,311 issued to Umemura et al. on July 25, 1995 teaches an ultrasound therapeutic system provided with an ultrasound transmitter having a focusing mechanism and a plurality of groups of ultrasound transmitters/receivers, each of which has a controllable directivity, each of the transmitters/receivers is constructed so as to be able to receive both echo of pulse-shaped ultrasound transmitted by itself and even order harmonic signals of the ultrasound transmitted by the transmitter, and a plurality of two-dimensional pulse echographical images constructed by ultrasound signals obtained by transmitting/receiving beams, while controlling the directivity of the beam emitted by each of the plurality of groups of ultrasound transmitters/receivers and a plurality of images indicating orientation and intensity, in which an even order harmonic wave signal due to the ultrasound transmitted by the transmitter is received by each of the plurality of groups of ultrasound transmitters/receivers, are displayed, superimposed on each other.

Yet still another example, U.S. Patent No. 5,498,236 issued to Dubrul et al. on March 12, 1996 teaches a catheter suitable for introduction into a tubular tissue for dissolving blockages in such tissue. The catheter is particularly useful for removing thrombi within blood vessels. In accordance with the preferred embodiments, a combination of vibrating motion and injection of a lysing agent is utilized to break up blockages in vessels. The vessels may be veins, arteries, ducts, intestines, or any lumen within the body that may become blocked from the material flowing through it. As a particular example, dissolution of vascular thrombi is facilitated by advancing a catheter through the occluded vessel with the catheter causing a vibrating stirring action in and around the thrombus, usually in combination with the dispensing of a thrombotic agent, such as urokinase into the thrombus. The catheter has an inflatable or expandable member near the distal tip that when inflated or expanded prevents the passage of dislodged thrombus around the catheter. The dislodged portions of the thrombus are directed through a perfusion channel in the catheter where they are removed by filtration apparatus housed within the perfusion channel before the blood exits the tip of the catheter. Catheters allowing both low frequency (1-1000 Hz) vibratory motion and delivery of such agents to a blockage and a method for using such catheters are disclosed.

Still yet another example, U.S. Patent No. 5,501,655 issued to Rolt et al. on March 26, 1996 teaches an ultrasound hyperthermia applicator suitable for medical hyperthermia treatment and a method for using it. The applicator includes two ultrasound sources producing focused ultrasound beams of frequencies *f₀* and *f₁*. An aiming device directs the two ultrasound beams so that they cross each other confocally at the target. A controller activates the two ultrasound sources so that the target is simultaneously irradiated by the two focused ultrasound beams. The two ultrasound sources provide acoustic energy sufficient to cause sufficient intermodulation products to be produced at the target as a result of the interaction of the two ultrasound beams. The intermodulation products are absorbed by the target to enhance heating of the target. In preferred embodiments, the ultrasound sources include a pair of signal generators for producing gated ultrasound output signals driving single crystal ultrasound transducers. In other embodiments, the ultrasound sources include a pair of phased array ultrasound transducers for generating two separate ultrasound beams. An aiming device is provided for electronically steering and focusing the two ultrasound beams so that they cross each other confocally at the target. Further embodiments employ pluralities of transducers, arrays, or both.

Yet still another example, U.S. Patent No. 5,503,150 issued to Evans on April 2, 1996 teaches a method and apparatus for noninvasively locating and heating a volume of tissue, specifically a cancerous tumor. The method includes placing a bolus in contact with the patient and substantially around an area of interest including the volume of tissue, placing an array of antennas on the bolus and substantially around the area of interest, imaging the area of interest, selecting an approximate center of the volume of tissue on the initial image, determining approximate amplitudes and phases for the antennas, energizing each element at respective appropriate amplitudes and phases to heat the volume of tissue, imaging respectively the area of interest to create subsequent images, and subtracting the initial image from the subsequent images to determine temperature changes in the area of interest.

Still yet another example, U.S. Patent No. 5,524,625 issued to Okazaki et al. on June 11, 1996 teaches a shock wave generating system capable of forming a wide concretion-disintegrating region by energizing ring-shaped transducers and a hyperthermia curing system. A width of a focused region synthesized from a plurality of focal points formed by a plurality of shock waves is varied by properly controlling delay times and/or drive voltages for a plurality of ring-shaped piezoelectric transducer elements. The shock wave generating system includes a shock wave generating unit having a plurality of shock wave generating elements and a driving unit for separately driving the plurality of shock wave generating elements by controlling at least delay times to produce a plurality of shock waves in a manner that a dimension of a focused region synthesized from a plurality of different focal points formed by the plurality of shock waves is varied in accordance with a dimension of a concretion to be disintegrated present in a biological body under medical examination.

Yet still another example, U.S. Patent No. 5,529,572 issued to Spector on June 25, 1996 teaches a method and apparatus for increasing the density and strength of bone, particularly for preventing or treating osteoporosis, by subjecting the bone to unfocussed compressional shock waves.

Still yet another example, U.S. Patent No. 5,542,906 issued to Herrmann et al. on August 6, 1996 teaches a therapy apparatus having a source of acoustic waves generating acoustic waves focused onto a focus and an X-ray locating apparatus with which the subject to be treated can be irradiated from different directions. The central ray of the locating apparatus assumes a first direction for a first irradiation direction and a second direction for a second irradiation direction. The apparatus has a positioning system with which the subject to be treated and the focus can be adjusted relative to one another. The region to be treated and the focus are adjustable relative to one another by synchronous actuation of the positioning system in two adjustment directions for at least one irradiation direction. The adjustment taking place in a direction proceeding parallel to the direction of the central ray belonging to the other irradiation direction.

Yet still another example, U.S. Patent No. 5,549,544 issued to Young et al. on August 27, 1996 teaches apparatus including a piezoelectric vibrator adapted to generate ultrasonic energy transmitted through an output section to a plastics head. The shape of the head may be varied to suit whichever part of a body on which it is to be used. The material and shape of the head is chosen to allow accurate control of frequency and amplitude of the ultrasonic energy.

Still yet another example, U.S. Patent No. 5,558,623 issued to Cody on September 24, 1996 teaches a therapeutic ultrasonic device transmitting multiple ultrasonic frequencies through one ultrasonic applicator. The applicator includes a handle, two diaphragms connected to one end of the handle with each diaphragm having an application face directed away from the handle and a rear face directed into the handle so that the application faces may be independently applied to a patient during therapy, and at least two piezoelectric crystals. A piezoelectric crystal is connected to the rear face of each diaphragm for converting periodic electrical energy into ultrasonic energy and transmitting the ultrasonic energy through the diaphragm to which the crystal is connected independently of the other diaphragm. An excitation source is provided for independently applying a periodic electric field of selectable frequency across a crystal in order to select the crystal to receive the periodic electric field and to select the ultrasonic frequency transmitted through the diaphragm to which the selected crystal is connected.

Yet still another example, U.S. Patent No. 5,713,848 issued to Dubrul et al. on February 3, 1998 teaches a catheter suitable for introduction into a tubular tissue for dissolving blockages in such tissue. The catheter is particularly useful for removing thrombi within blood vessels. In accordance with the preferred embodiments, a combination of vibrating motion and injection of a lysing agent is utilized to break up blockage in vessels. The vessels may be veins, arteries, ducts, intestines, or any lumen within the body that may become blocked from the material flowing through it. As a particular example, dissolution of vascular thrombi is facilitated by advancing a catheter through the occluded vessel. The catheter causes a vibrating, stirring action in and around the thrombus usually in combination with the dispensing of a thrombolytic agent, such as urokinase into the thrombus. The catheter has an inflatable or expandable member near the distal tip that when inflated or expanded prevents the passage of dislodged thrombus around the catheter. The dislodged portions of the thrombus are directed through a profusion channel in the catheter where they are removed by filtration apparatus housed within the perfusion channel before the blood exits the tip of the catheter. Catheters allowing both low frequency (1-1000 Hz) vibratory motion and delivery of such agents to a blockage and a method for using such catheters are disclosed.

It is apparent that numerous other innovations for wave treatments have been provided in the prior art that are adapted to be used. Furthermore, even though these other innovations may be suitable for the specific individual purposes to which they address, however, they would not be suitable for the purposes of the present invention as heretofore described.

### Summary of the Invention

Accordingly, an object of the present invention is to provide a neoplasm cell destruction device that avoids the disadvantages of the prior art.

Briefly stated, another object of the present invention is to provide a neoplasm cell destruction device. The device includes at least one signal generator and at least one transducer. The at least one transducer is driven by the at least one signal generator, and produces sound waves. The sound waves impact upon a neoplastic target to damage, and ultimately destruct, the neoplastic target.

The novel features considered characteristic of the present invention are set forth in the appended claims. The invention itself, however, both as to its construction and its method of operation, together with additional objects and advantages thereof will be best understood from the following description when read and understood in connection with the accompanying drawing.

### Brief Description of the Drawing

The figures of the drawing are briefly described as follows:
- **FIGURES 1A-1B**: are a block diagram of the neoplasm cell destruction device of the present invention; and
- **FIGURES 2A-2G**: are a flow chart of the method of operation of the neoplasm cell destruction device of the present invention.

### Detailed Description of the Preferred Embodiment

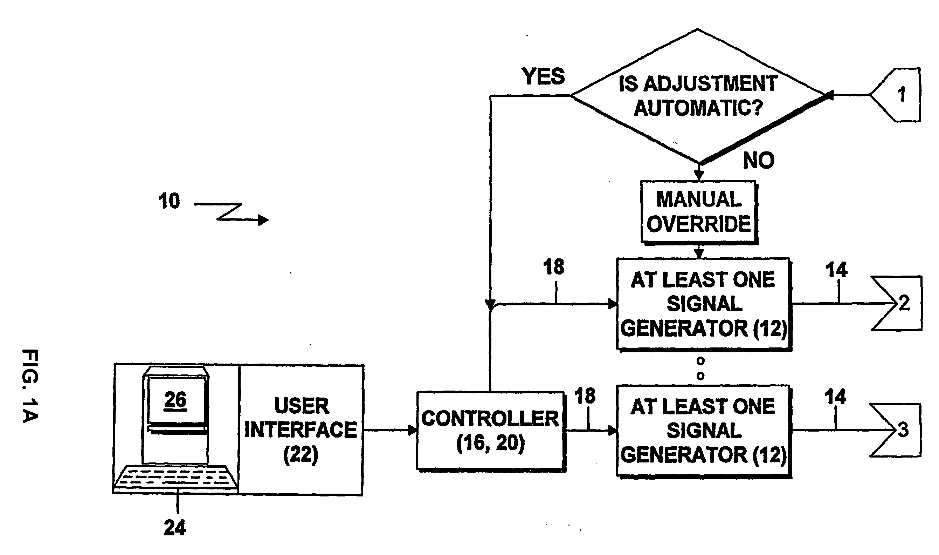

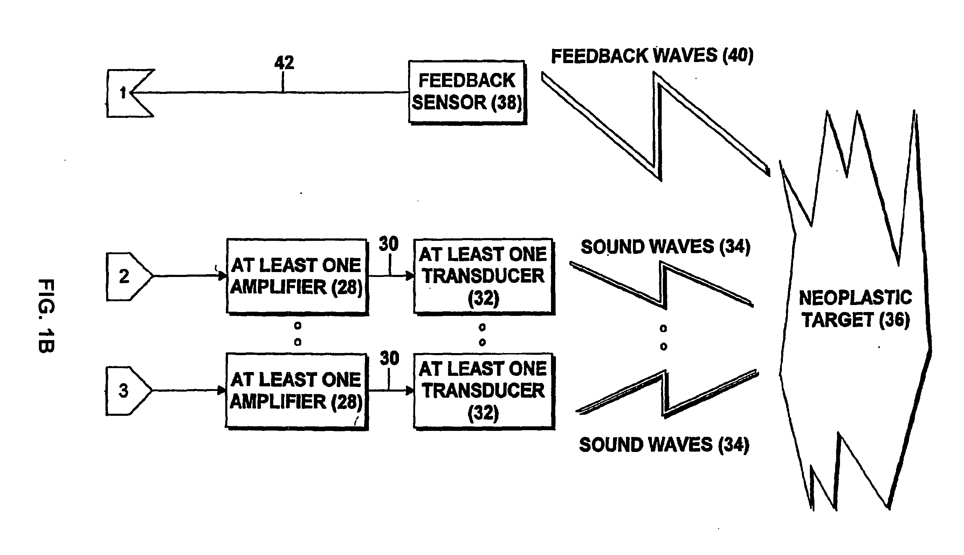

Referring now to the figures, in which like numerals indicate like parts, and particularly to **FIGURES 1A** to **1B**, which is a block diagram of the neoplasm cell destruction device of the present invention, the neoplasm cell destruction device of the present invention is shown generally at **10.**

The neoplasm cell destruction device **10** comprises at least one signal generator **12.** The at least one signal generator **12** generates signals **14.**

The neoplasm cell destruction device **10** further comprises a controller **16.** The controller **16** is in communication with, and generates timing and control signals **18** to selectively activate, the at least one signal generator **12,** and can be, *inter alia*, a microprocessor **20.**

The neoplasm cell destruction device **10** further comprises a user interface **22.** The user interface **22** is in communication with the controller **16,** and can be, *inter alia*, a keyboard **24** and/or a display **26.**

The neoplasm cell destruction device **10** further comprises at least one amplifier **28.** The at least one amplifier **28** is in communication with the at least one signal generator **12,** and amplifies the signals **14** generated thereby to produce amplified signals **30.**

The neoplasm cell destruction **10** further comprises at least one transducer **32.** The at least one transducer **32** is in communication with the at least one amplifier **28,** and is driven by the amplified signals **30** to produce sound waves having frequencies in a range of 20 to 20,000 Hz. The sound waves **34** - which due to lower energy than prior treatment sources reduces lethality to surrounding healthy cells - is impacted upon a neoplastic target **36** to damage, and ultimately destruct, the neoplastic target **36,** and can form interference waves providing synergistic effect.

The neoplastic target **36** exhibits several resonant frequencies corresponding, for example, to distortion of the cell wall, distortion of the nucleus, etc. As with all objects, if the neoplastic target **36** is impacted with energy at a predetermined frequency, the portion of the input energy converted into mechanical energy, *i.e.* motion, will be significantly enhanced if the input frequency is at one of the resonant frequencies of the neoplastic target **36.** *See* U.S. Patent Number 4,315,514 to Drewes et al. ("Drewes") at col. 1, lines 59-66. Drewes teaches how to determine resonant frequencies, and as such, is incorporated herein by reference thereto.

The neoplasm cell destruction **10** further comprises a feedback sensor **38.** The feedback sensor **38** is in communication with the controller **16.** The feedback sensor **38** receives feedback waves **40** emanating from the neoplastic target **36** when the neoplastic target **36** is impacted upon by the sound waves **34,** and generates feedback signals **42** in response thereto, which is received by the controller **16,** which in turn continually compares the feedback signals **42** to the sound waves **34** and automatically adjusts the at least one signal generator **12** accordingly until the sound waves **34** are at a resonant frequency of the neoplastic target **36** so as to maximize damage to the neoplastic target **36.**

By utilizing the feedback sensor **38,** one need not know in advance the resonant frequency to be used. The feedback sensor **38** receives the feedback waves **40** emanating from the neoplastic target **36** when the neoplastic target **36** is impacted upon by the sound waves **34,** and generates the feedback signals **42** in response thereto, which is received by the controller **16,** which in turn continually compares the feedback signals **42** to the sound waves **34** and automatically adjusts the at least one signal generator **12** accordingly until the sound waves **34** are at a resonant frequency of the neoplastic target **36.**

It is to be understood that the controller **16** can be manually overridden. If so, the feedback signals **42** from the feedback sensor **38** would then go directly to the at least one signal generator **12,** which would be manually adjusted accordingly until the sound waves **34** are at a resonant frequency of the neoplastic target **36** so as to maximize damage to the neoplastic target **36.**

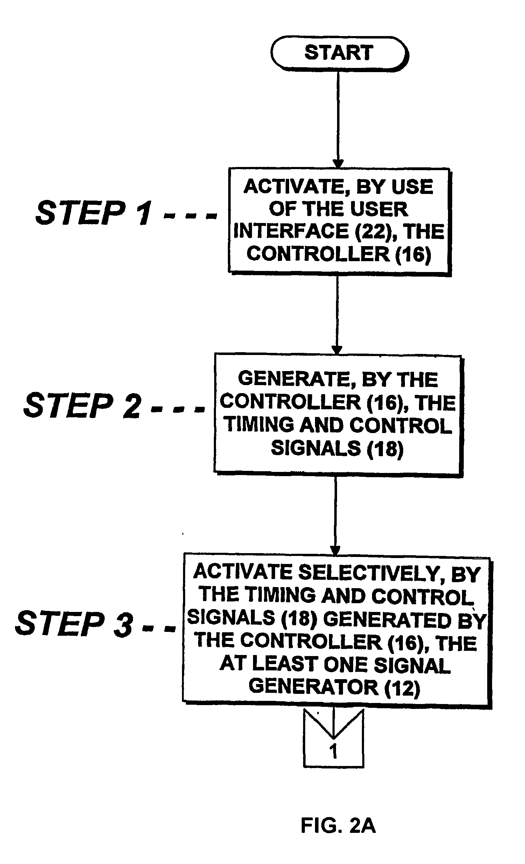

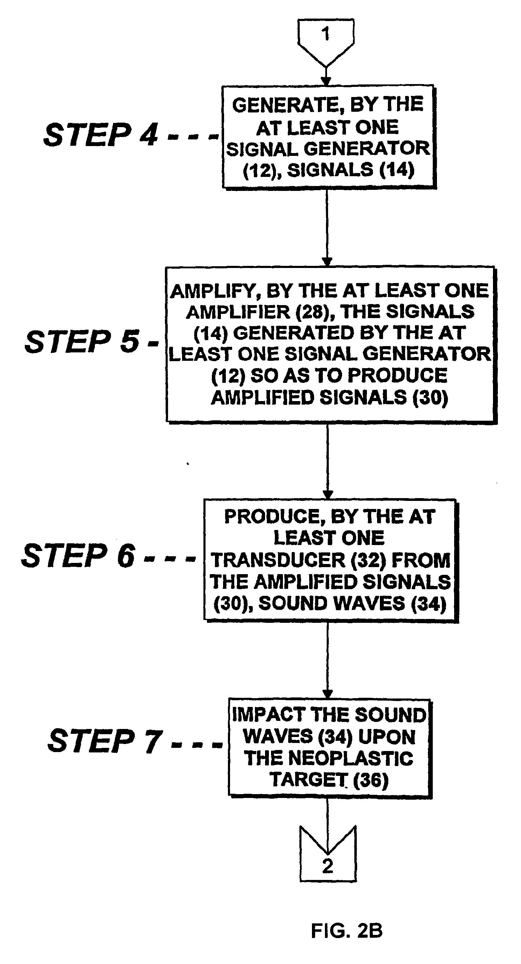

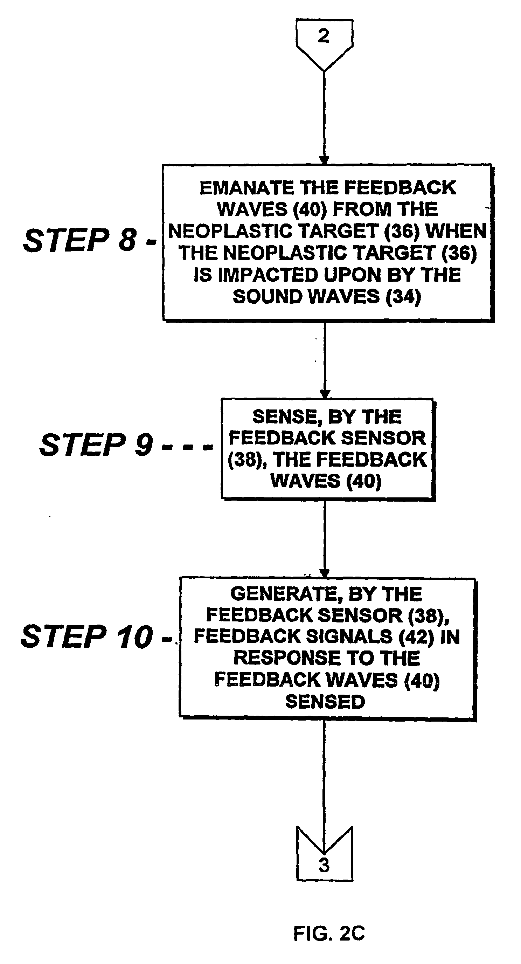

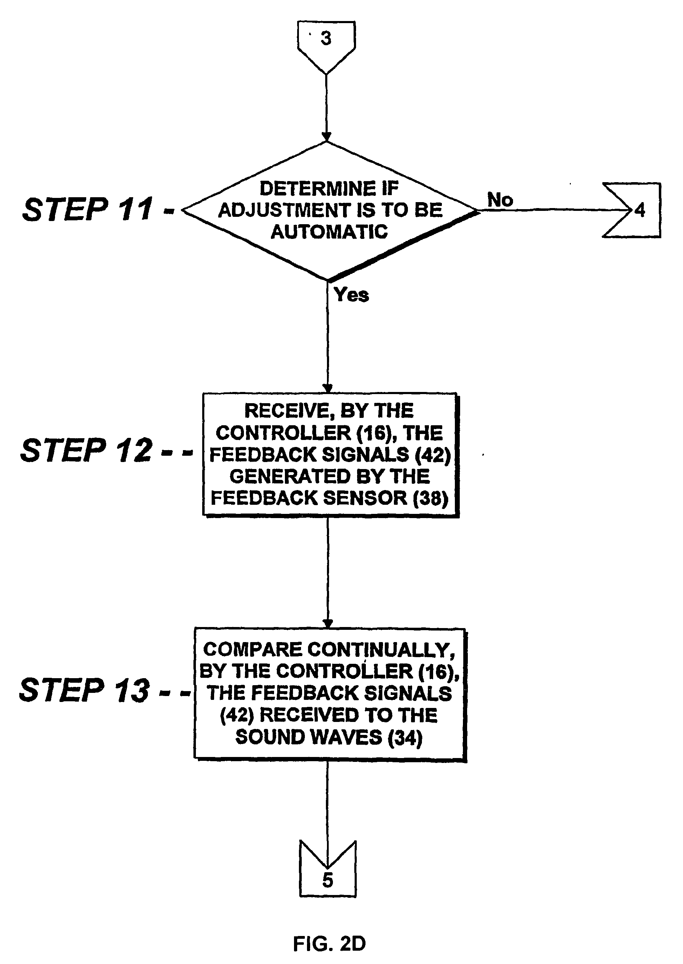

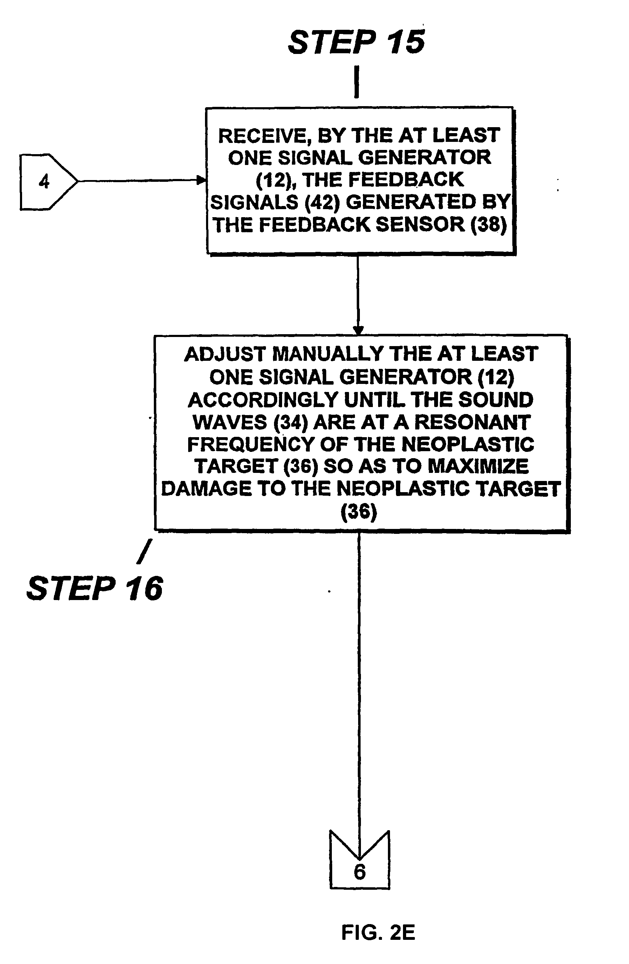

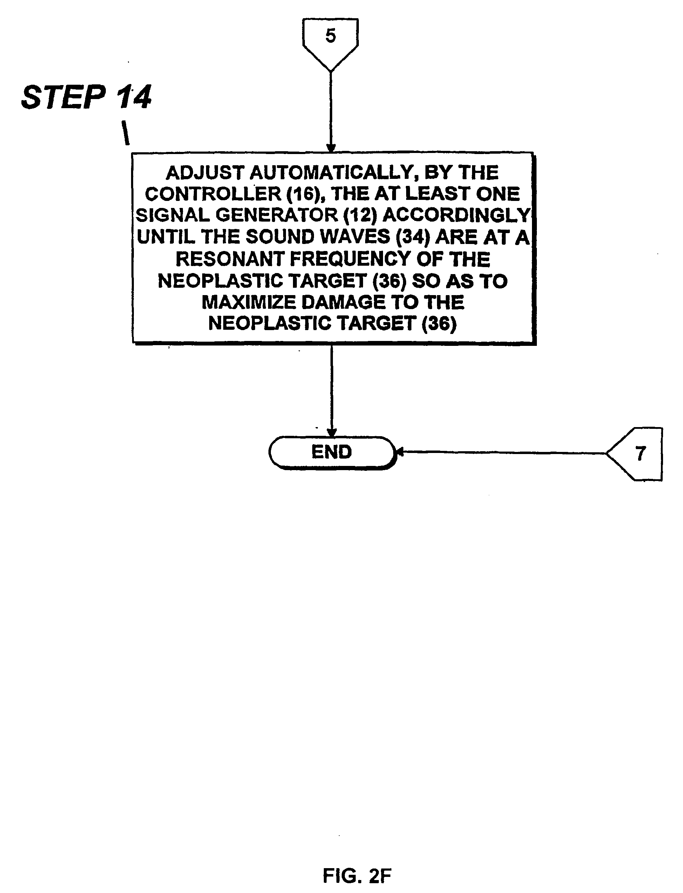

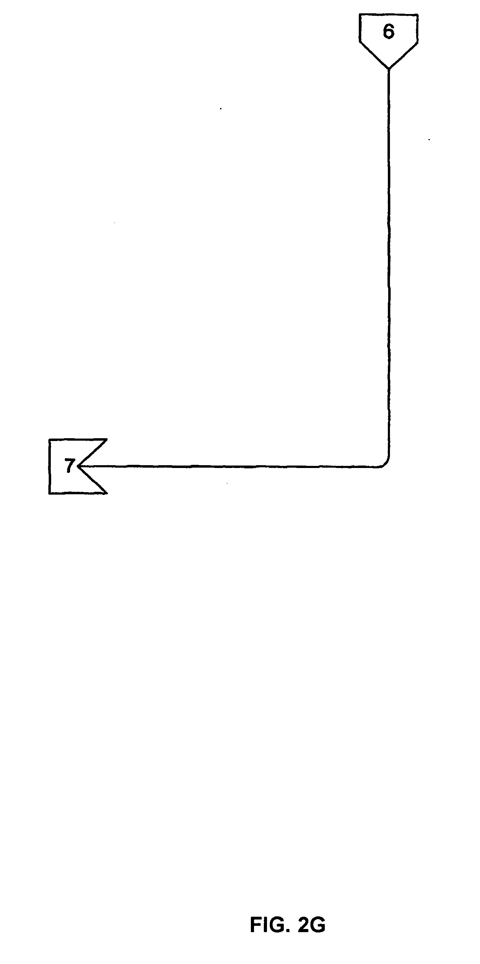

The method of operation of the neoplasm cell destruction device **10** can best be seen in **FIGURES 2A** to **2G****,** which is a flow chart of the method of operation of the present invention, and as such, will be discussed with reference thereto.
- ***STEP* 1:**: Activate, by use of the user interface **22,** the controller **16.**
- ***STEP* 2:**: Generate, by the controller **16,** timing and control signals **18.**
- ***STEP* 3:**: Activate selectively, by the timing and controls signals **18** generated by the controller **16,** the at least one signal generator **12.**
- ***STEP* 4:**: Generate, by the at least one signal generator **12,** signals **14.**
- ***STEP* 5:**: Amplify, by the at least one amplifier **28,** the signals **14** generated by the at least one signal generator **12** to produce amplified signals **30.**
- ***STEP* 6:**: Produce, by the at least one transducer **32** from the amplified signals **30,** sound waves **34.**
- ***STEP* 7:**: Impact the sound waves **34** upon the neoplastic target **36.**
- ***STEP* 8:**: Emanate the feedback waves **40** from the neoplastic target **36** when the neoplastic target **36** is impacted upon by the sound waves **34.**
- ***STEP* 9:**: Sense, by the feedback sensor **38,** the feedback waves **40.**
- ***STEP 10:***: Generate, by the feedback sensor **38,** a feedback signals **42** in response to the feedback waves **40** sensed.
- ***STEP* 11:**: Determine if adjustment is to be automatic.
- ***STEP* 12:**: Receive, by the controller **16,** the feedback signals **42** generated by the feedback sensor **38,** if answer to ***STEP* 11** is yes.
- ***STEP* 13:**: Compare continually, by the controller **16,** the feedback signals **42** received to the sound waves **34.**
- ***STEP* 14:**: Adjust automatically, by the controller **16,** the at least one signal generator **12** accordingly until the sound waves **34** are at a resonant frequency of the neoplastic target **36** so as to maximize damage to the neoplastic target **36.**
- ***STEP* 15:**: Receive, by the at least one signal generator **12,** the feedback signals **42** generated by the feedback sensor **38,** if answer to ***STEP* 11** is no.
- ***STEP* 16:**: Adjust manually the at least one signal generator **12** accordingly until the sound waves **34** are at a resonant frequency of the neoplastic target **36** so as to maximize damage to the neoplastic target **36.**

It will be understood that each of the elements described above or two or more together may also find a useful application in other types differing from the types described above.

While the invention has been illustrated and described as embodied in a neoplasm cell destruction device, however, it is not limited to the details shown, since it will be understood that various omissions, modifications, substitutions, and changes in the forms and details of the device can be made by those skilled in the art without departing in any way from the scope of the present invention.

Without further analysis the foregoing will so fully reveal the gist of the present invention that others can by applying current knowledge readily adapt it for various applications without omitting features that from the standpoint of prior art fairly constitute characteristics of the present invention.

## Claims

1. A neoplasm cell destruction device, comprising: at least one signal generator (12); and at least one transducer (32), wherein said at least one transducer (32) is driven by said at least one signal generator(12), wherein said at least one transducer produces sound waves (34);
said sound waves (34) are impacted upon a neoplastic target (36) to damage and ultimately destruct the neoplastic target (36), whereas the device further comprising a controller (16), which is in communication with said at least one signal generator (12) and generates timing and control signals to selectively activate said at least one signal generator, and at least one amplifier (28); which amplifies said signals generated by said at least one signal generator (12) so as to produce amplified signals (30), and wherein said at least one transducer (32) is driven by said amplified signals (30), and wherein said sound waves have frequencies in a range of 20-20,000 Hz.
**Characterized in that** a feedback sensor (38), which is in communication with said controller (16), receives feedback sound waves (40) emanating from the neoplastic target (36), when the neoplastic target is impacted upon by said sound waves (34) and generates feedback signals (42) in response thereto that are received by said controller (16), that in turn continually compares the feedback signals (42) received to said sound waves (40) and automatically adjusts said at least one signal generator (12) accordingly until said sound waves (40) are at a resonant frequency of the neoplastic target so as to maximize damage to the neoplastic target.

2. The device as defined in claim 1, **characterized in that** said sound waves form interference waves providing synergistic effect.

3. The device as defined in claim 1, **characterized in that** said controller is a microprocessor.

4. The device as defined in claim 1, **characterized in that** the device further comprising a user interface; and wherein said user interface is in communication with said controller.

5. The device as defined in claim 4, **characterized in that** said user interface is at least one of a keyboard and a display.

6. The device as defined in claim 1, **characterized in that** the device further comprising a feedback sensor; therein said feedback sensor is in communication with said at least one signal generator; and wherein said feedback sensor receives feedback waves emanating from the neoplastic target when the neoplastic target is impacted upon by said sound waves and generates feedback signals- in response thereto that are received by said at least one signal generator that in turn is manually adjusted accordingly until said sound waves are at a resonant frequency of the neoplastic target so as to maximize damage to the neoplastic target.

## Patentansprüche

1. Vorrichtung zur Zerstörung von Neoplasien, umfassend wenigstens einen Signalgenerator (12); und wenigstens einen Schallgeber (32), wobei dieser wenigstens eine Schallgeber (32) durch diesen wenigstens einen Signalgenerator (12) angetrieben ist, wobei dieser wenigstens eine Schallgeber Schallwellen (34) produziert; dass zur Beschädigung und schliesslich zur Zerstörung der neoplastischen Stelle (36) diese Schallwellen (34) die neoplastische Stelle (36) beaufschlagen, wobei die Vorrichtung des weiteren einen Regler (16) aufweist, welcher in Verbindung mit dem wenigstens einen Signalgenerator (12) steht und den Takt und Steuersignale zur selektiven Aktivierung dieses wenigstens einen Signalgenerators erzeugt, und wenigstens ein Verstärker (28); welcher diese vom wenigstens einen Signalgenerator (12) erzeugten Signale verstärkt, um damit verstärkte Signale (30) zu erzeugen, und wobei dieser wenigstens eine Schallgeber (32) durch diese verstärkten Signale (30) betrieben ist, und wobei diese Schallwellen im Frequenzbereich von 20 - 20'000 Hz liegen, **dadurch gekennzeichnet, dass**
ein Rückmeldesensor (38) vorgesehen ist, welcher in Verbindung mit diesem Regler (16) ist, Rückmeldeschallwellen (40) ausgehend von der neoplastischen Stelle (36) empfängt, wenn die neoplastische Stelle mit diesen Schallwellen (34) beaufschlagt ist, und Rückmeldesignale (42) erzeugt als Rückmeldung dazu, wobei diese von diesem Regler (16) empfangen werden, dass im Wechsel kontinuierlich durch die Schallwellen (40) empfangenen Rückmeldesignale (42) verglichen und diesen wenigstens einen Signalgenerator (12) automatisch einstellt entsprechend bis diese Schallwellen (40) in einer Resonanzfrequenz der neoplastischen Stelle sind, um so die Beschädigung der neoplastischen Stelle zu maximieren.

2. Vorrichtung nach Anspruch 1, **dadurch gekennzeichnet, dass** diese Schallwellen Empfangsstörungen zur Erzielung eines synergistischen Effekts bilden.

3. Vorrichtung nach Anspruch 1, **dadurch gekennzeichnet, dass** dieser Regler ein Mikroprozessor ist.

4. Vorrichtung nach Anspruch 1, **dadurch gekennzeichnet, dass** die Vorrichtung zudem ein Anwender-Interface umfasst; und dass dieses Anwender-Interface in Verbindung mit diesem Regler steht.

5. Vorrichtung nach Anspruch 4, **dadurch gekennzeichnet, dass** dieses Anwender-Interface wenigstens eines aus einer Tastatur und einem Bildschirm ist.

6. Vorrichtung nach Anspruch 1, **dadurch gekennzeichnet, dass** die Vorrichtung zudem einen Rückmeldesensor aufweist, wobei dieser Rückmeldesensor in Verbindung mit diesem wenigstens einen Signalgenerator ist, und wobei dieser Rückmeldesensor Rückmeldeschallwellen ausgehend von der neoplastischen Stelle empfängt, wenn die neoplastische Stelle mit diesen Schallwellen beaufschlagt ist, und Rückmeldesignale erzeugt als Rückmeldung dazu, wobei diese von diesem Signalgenerator empfangen werden, dass im Wechsel manuell eingestellt entsprechend bis diese Schallwellen (40) in einer Resonanzfrequenz der neoplastischen Stelle sind, um so die Beschädigung der neoplastischen Stelle zu maximieren.

## Revendications

1. Dispositif de destruction de cellules néoplasiques, comprenant au moins un générateur de signal (12) ; et au moins un transducteur (32) dans lequel ledit au moins un transducteur (32) est piloté par ledit au moins un générateur de signal (12) et ledit au moins un transducteur produit des ondes sonores (34) ;
lesdites ondes sonores (34) percutant une cible néoplasique (36) afin d'endommager et finalement détruire la cible néoplasique (36), le dispositif comprenant en outre un organe de commande (16), qui est en communication avec ledit au moins un générateur de signal (12) et génère des signaux d'horloge et de commande pour activer sélectivement ledit au moins un générateur de signal, et au moins un amplificateur (28) ; qui amplifie lesdits signaux générés par ledit au moins un générateur de signal (12) afin de produire des signaux amplifiés (30), et ledit au moins un transducteur (32) étant piloté par lesdits signaux amplifiés, et lesdites ondes sonores ayant des fréquences dans la gamme de 20 à 20 000 Hz,
**caractérisé en ce qu'**un capteur de retour (38), qui est en communication avec ledit organe de commande (16), reçoit des ondes sonores de retour (40) provenant de la cible néoplasique (36), lorsque la cible néoplasique est percutée par lesdites ondes sonores (34), et génère en réponse des signaux de retour (42) qui sont reçus par ledit organe de commande (16), qui compare de son côté en continu les signaux de retour (42) reçus avec lesdites ondes sonores (40) et règle automatiquement ledit au moins un générateur de signal (12) en conséquence jusqu'à ce que lesdites ondes sonores (40) soient à une fréquence de résonance de la cible néoplasique de manière à maximiser l'endommagement de la cible néoplasique.

2. Dispositif selon la revendication 1, **caractérisé en ce que** lesdites ondes sonores forment des ondes d'interférence produisant un effet synergétique.

3. Dispositif selon la revendication 1, **caractérisé en ce que** ledit organe de commande est un microprocesseur.

4. Dispositif selon la revendication 1, **caractérisé en ce que** le dispositif comprend en outre une interface utilisateur ; et dans lequel ladite interface utilisateur est en communication avec ledit organe de commande.

5. Dispositif selon la revendication 4, **caractérisé en ce que** ladite interface utilisateur est au moins un parmi un clavier et un dispositif d'affichage.

6. Dispositif selon la revendication 1, **caractérisé en ce que** le dispositif comprend en outre un capteur de retour ; et dans lequel ledit capteur de retour est en communication avec ledit au moins un générateur de signal ; et ledit capteur de retour recevant des ondes de retour provenant de la cible néoplasique lorsque la cible néoplasique est percutée par lesdites ondes sonores et générant en réponse des signaux de retour qui sont reçus par ledit au moins un générateur de signal qui est de son côté réglé en conséquence manuellement jusqu'à ce que lesdites ondes sonores soient à une fréquence de résonance de la cible néoplasique de manière à maximiser l'endommagement de la cible néoplasique.
